# EUROPEAN PATENT APPLICATION

(11) **EP 1 749 919 A1**
(43) Date of publication of application: **07.02.2007**
(21) Application number: 06015326.9
(22) Date of filing: 24.07.2006
(51) Int. Cl.: D06M 13/203, D06M 13/192, A61K 8/37

(54) **Sizing agent capable of neutralising odours or preventing odours from forming and process related thereto**

(30) Priority: 03.08.2005 IT MI20051526
(71) Applicant: De Leuriks B.V., 1015 BV Amsterdam (NL)
(72) Inventor: Varana, Ferruccio, 20037 Paderno Dugnano (MI) (IT)
(74) Representative: La Ciura, Salvatore

(57) **Abstract**

This invention proposes a sizing agent capable of neutralising odours on clothes and garments, which contains a proper amount of zinc ricinoleate, which acts as odour neutraliser and of triethyl citrate, capable of preventing such odours from forming.
In particular, the sizing agent provides for the following formulation, in which the different components are expressed as weight:
- Zinc ricinoleate from 0.05% to 3%
- Hydrogenated polyethossilate castor oil with 40 moles of ethylene oxide from 0.1% to 3%
- Dimethylpolysiloxane (silicone) from 0.05% to 2%
- Perfume from 0.02% to 3%
- Triethyl citrate from 0.1% to 4%
- Vegetable starch from 0.5% to 5%
- Preservatives q.s.
- Demineralised water q.s. at 100
- Citric acid q.s. at pH6

The invention concerns also the method for producing the sizing agent.

## Description

This invention proposes a sizing agent having a particular formulation capable of neutralising odours on clothes and garments with which the sizing agent is used during ironing, as well as capable of preventing such odours from forming.

More particularly, the sizing agent according to the invention contains a proper amount of zinc ricinoleate, which acts as odour neutraliser and of triethyl citrate, capable of preventing such odours from forming.

The invention concerns also the method for producing the sizing agent.

As is known the sizing agents are composed of particular pastes or fluid Solutions made of various substances, which are applied to a cloth in order to give it particular appearance properties.

The sizing agents can include softeners, containing for example fats, oils, waxes, wool-fat, etc., thickeners, generally starch-, dextrin-, glue-based or others.

The most commonly used household sizing agents are starch-based agents, which are used to give a higher stiffness to clothes and to remove creases from them.

This invention falls into this sector.

The problem solved by the invention results from the ascertainment of the fact that quite often in the garments and clothes, soon after having worn them, bad odours develop, as a result, for example, of sweating.

Different products capable of fighting bad odours are known, but they consist of kind of "scented" products, i.e. products containing a certain amount of fragrant essences capable of overlapping and covering the cloth odours.

It is plain that the aforesaid products do not represent the ideal solution, both because not always a person wishes to make use of perfumes, and because these products quickly evaporate and therefore their effect decrease, and because not always the perfume when mixes with the substances which develop bad odours gives the desired effect.

This problem is now overcome by this invention, which proposes a sizing agent containing substances which do not act by covering the odours, but which, on the one hand, interact with the scented substances molecules, by immobilising them and neutralising their odours, and on the other hand interact with the bacteria which cause their formation, inhibiting it.

This invention will be better explained by the following detailed example, which refers to a formulation of the sizing agent according to the invention.

In compliance with the invention, a possible formulation of odour neutralising sizing agent is the one shown hereafter, in which the percentages of the different components are expressed as weight:
- Zinc ricinoleate from 0.05% to 3%
- Hydrogenated polyethossilate castor oil with 40 moles of ethylene oxide from 0.1% to 3%
- Dimethylpolysiloxane (silicone) from 0.05% to 2%
- Perfume from 0.02% to 3%
- Triethyl citrate from 0.1% to 4%
- Vegetable starch from 0.5% to 5%
- Preservatives q.s.
- Demineralised water q.s. at 100
- Citric acid q.s. at pH6

The zinc ricinoleate acts as odour neutraliser.

In particular the said component is composed of one long-chain molecule which combines with the molecules of the fragrant substances making them heavier and, practically, immobilizing them and thus neutralising the odours.

An example of zinc ricinoleate is the one commercialised by the company Goldschmidt under "Tego Sorb conc. 50°" trademark.

The silicone, for example commercialised under HV-495 acronym by the company Dow Corning, acts as lubricant to make ironing easier, while the vegetable starch, for example tapioca flour commercialised by the company Ettilinger, represents the actual sizing agent.

The hydrogenated castor oil, for example the one commercialised by the company Basf under Cremophor RG 40 trade name, is a solubilizing agent used to keep in solution the zinc ricinoleate.

The triethyl citrate serves as antibacterial agent and acts on the bacteria which, by digesting the substances which form the odours, for example the substances present in sweat, cause their fermentation and the resulting odour formation.

A type of triethyl citrate is represented, by way of example, by the one commercialised under Citrofol A1 trade name by the company Jungbunziauer.

As a preservative, a suitable product can be represented by the one commercialised under Kathon CG trade name by the company Rohm & Haas.

The sizing agent thus obtained guarantees the neutralisation of odours still present in the cloth and prevents new odours from forming.

The process for preparing the sizing agent is the following:

Load a portion of water used in the formulation and heat to 50°C; then disperse the tapioca starch under stirring; keep for twenty minutes and gradually increase the temperature firstly to 60°-70°C, then up to 90°C, keeping the mixture under stirring until the starch swells up and disperses.

Let it cool down, while verifying that the starch remains in suspension.

Then mix the zinc ricinoleate with hydrogenated polyethossilate castor oil.

Apart, dissolve the dimethylpolysiloxane in water and add it to the mixture.

Emulsify apart the hydrogenated polyethossilate castor oil, the triethyl citrate, the zinc ricinoleate and the perfume and add them to water.

Finally, add the preservative and adjust the mixture with citric acid until it reaches a pH value equal to 5-6.

Once the mixture is filtered, the product is ready.

The sizing agent appears under fluid form and can be easily applied to the clothes, for example by spraying. A skilled in the art may make changes and different versions, which must be considered all included within the competence of this invention.

## Claims

1. Odour neutralizer sizing agent, **characterised in that** it comprises a proper amount of zinc ricinoleate and triethyl citrate.

2. Odour neutralizer sizing agent according to claim 1, **characterised in that** it also comprises a proper amount of hydrogenated polyethossilate castor oil as solubilizing agent of the zinc ricinoleate.

3. Sizing agent according to claim 3, in which the starch is composed of vegetable starch.

4. Sizing agent according to claim 3, in which the vegetable starch is tapioca starch.

5. Odour neutralizer sizing agent according to any of the preceding claims, **characterised in that** it comprises the following formulation, in which the different components are expressed as weight:
- Zinc ricinoleate from 0.05% to 3%
- Hydrogenated polyethossilate castor oil with 40 moles of ethylene oxide from 0.1% to 3%
- Dimethylpolysiloxane (silicone) from 0.05% to 2%
- Perfume from 0.02% to 3%
- Triethyl citrate from 0.1% to 4%
- Vegetable starch from 0.5% to 5%
- Preservatives q.s.
- Demineralised water q.s. at 100
- Citric acid q.s. at pH6

6. Process for producing a neutralizer sizing agent according to any of the preceding claims, **characterised in that** it comprises the following phases:
- dispersion under stirring of tapioca starch in water at 50°C;
- increase of temperature up to 90°C, while keeping under stirring up to complete swelling and dispersion of the starch;
- mixture of zinc ricinoleate and hydrogenated polyethossilate castor oil;
- solution in water of dimethylpolysiloxane and addition of it to the mixture;
- separate emulsion of hydrogenated polyethossilate castor oil, triethyl citrate, zinc ricinoleate and perfume and addition to the mixture;
- pH adjustment by addition of citric acid, up to pH 5-6.
